# EUROPEAN PATENT SPECIFICATION

(11) **EP 2 470 175 B1**
(45) Date of publication and mention of the grant of the patent: **23.03.2016**
(21) Application number: 10759692.6
(22) Date of filing: 27.08.2010
(51) Int. Cl.: A61K 31/352, A61P 31/00

(54) **FULVIC ACID COMPOSITIONS AND THEIR USE**
FULVINSÄUREZUSAMMENSETZUNGEN UND IHRE VERWENDUNG
COMPOSITIONS À BASE D ACIDE FULVIQUE ET LEUR UTILISATION

(30) Priority: 27.08.2009 GB 0914966
(43) Date of publication of application: 04.07.2012
(73) Proprietor: Natracine UK Limited, Macclesfield, Cheshire SK10 4QH (GB)
(72) Inventor: LEIVERS, Stephen, William, Norfolk NR18 0EA (GB)
(74) Representative: Kremer, Simon Mark
(86) International application number: PCT/GB2010/001641
(87) International publication number: WO 2011/023970

(56) References cited:
- WO-A1-00/19999
- WO-A1-2007/102813
- WO-A1-2009/147635
- WO-A2-2007/125492
- WILSON M: "Susceptibility of oral bacterial biofilms to antimicrobial agents.", JOURNAL OF MEDICAL MICROBIOLOGY FEB 1996, vol. 44, no. 2, February 1996 (1996-02), pages 79-87, ISSN: 0022-2615
- GALLOWAY DAVID J ET AL: "A new species of Placopsis (Agyriaceae : Ascomycota) from Antarctica", LICHENOLOGIST (LONDON), vol. 37, no. Part 4, 2005, pages 321-327, ISSN: 0024-2829
- ABDI-ALI A ET AL: "Bactericidal activity of various antibiotics against biofilm-producing Pseudomonas aeruginosa", INTERNATIONAL JOURNAL OF ANTIMICROBIAL AGENTS, vol. 27, no. 3, March 2006 (2006-03), pages 196-200, ISSN: 0924-8579
- NETT JENIEL E: "Future directions for anti-biofilm therapeutics targeting Candida", EXPERT REVIEW OF ANTI-INFECTIVE THERAPY, vol. 12, no. 3, March 2014 (2014-03), pages 375-382, ISSN: 1478-7210(print)

## Description

### FIELD OF INVENTION

This invention relates to a use of fulvic acid in the prevention and treatment of infection by oral pathogens.

### BACKGROUND OF THE INVENTION

Fulvic acid (FA) is a humic substance along with humic acid and humin that is formed during the decay of organic matter (MacCarthy et al., 1985). These substances are characterised on the basis of their solubility in water as a function of pH and FA is the fraction that is soluble in water under all pH conditions. In general, FA is also lower in molecular size and weight and lower in colour intensity than the humic acids.

Although soil and water naturally contain low levels of FA, this is difficult to isolate. Wet oxidation of bituminous coal, as described in US Patent No. 4,912,256 yields oxifulvic acids. Use of these oxifulvic acids for treatment of inflammation, acne, eczema, bacterial, fungal and viral infections has been described in US Patent Nos. 4,999,202 and 5,204,368 and International Patent Publication No. WO00/19999. However, oxifulvic acids contain high concentrations of heavy metals, including mercury, aluminium, chromium, lead and cadmium and are therefore not appropriate for use in medical, pharmaceutical and cosmetic preparations.

Carbohydrate sources such as saccharides including glucose, sucrose and fructose, starches and cellulose can also be treated by wet oxidation and produce a carbohydrate derived fulvic acid composition. This carbohydrate derived fulvic acid is suitable for use in medical, pharmaceutical and cosmetic preparations as it contains only a low content of the harmful elements. International Patent Publication No. WO2007/125492 describes the carbohydrate derived fulvic acid composition and a method for producing the composition (hereinafter referred to as CHD-FA).

### DISCLOSURE OF THE INVENTION

It has been found that fulvic acid as described and claimed in International Patent Publication No. WO 2007/125492 (CHD-FA), a salt or ester thereof is effective in preventing, treating or inhibiting infection by oral pathogens in mammals wherein the infection by an oral pathogen is in the form of an oral biofilm.

Thus, the invention provides, according to one aspect, CHD-FA, a salt or ester thereof for use in the prevention, treatment or inhibition of infection by oral pathogens in a mammal, which may be human or animal wherein the infection by an oral pathogen is in the form of an oral biofilm.

The CHD-FA, salt or ester thereof may be administered in an amount from about 8% to 60% v/v of CHD-FA in an oral formulation. Preferably, the amount is about 35% v/vof CHD-FA in an oral formulation.

The CHD-FA, salt or ester thereof can have any pH, from acid to basic. For example, the pH of the CHD-FA can be raised by converting the acid into a salt, such as the sodium or potassium salt. This may be achieved by adding a suitable hydroxide to the CHD-FA.

According to a further embodiment of the invention, there is provided a composition comprising CHD-FA, salt or ester thereof and a suitable carrier for oral administration.

The composition may be in the form of a mouthwash, toothpaste, denture cleanser, impregnated dental floss or dental tape, endodontic irrigant or the like. The CHD-FA, salt, ester or derivative thereof may be an active ingredient or an adjuvant. The composition may further comprise flavourants and the like.

Also described is a method of preventing, treating or inhibiting infection by oral pathogens in a mammal including the step of administering an effective amount of the CHD-FA, salt or ester thereof to the mammal wherein the infection by an oral pathogen is in the form of an oral biofilm.

The administration will be oral. For human administration, the CHD-FA, salt or ester thereof may be formulated into a form such as a mouthwash, toothpaste, denture cleanser, impregnated dental floss or dental tape, endodontic irrigant or the like. The CHD-FA, salt or ester thereof may be the active ingredient or an adjuvant. The formulated form may further comprise flavourants and the like.

### BRIEF DESCRIPTION OF THE DRAWINGS

**Figure 1** shows the effectiveness of CHD-FA in killing oral biofilms in comparison to other treatments i.e. Tea Tree Oil and the commercially available products, Oral B and Corsodyl.
**Figure 2** shows the relative lack of toxicity of CHD-FA when applied to the OKF6 cell line.
**Figure 3** shows the immuno-modulatory effect of CHD-FA on IL-6 and IL-8.
**Figure 4** shows the relative lack of toxicity of CHD-FA compared to Chlorhexadine, an ingredient commonly used to treat oral pathogens.

### DESCRIPTION OF PREFERRED EMBODIMENTS

CHD-FA, a salt or ester thereof for use in a method of preventing, treating or inhibiting infection by oral pathogens in mammals is described herein.

The optimal dose of the CHD-FA, a salt or ester thereof is an amount of about 35% of CHD-FA in an oral formulation maintained in the mouth for about 60 seconds. However a range of from about 8% to about 60% v/v CHD-FA in an oral formulation maintained in the mouth from about 30 seconds to about 60 seconds could also be used.

The CHD-FA, salt or ester thereof can have any pH, from acid to basic. For example, the pH of the CHD-FA can be raised by converting the acid into a salt, such as the sodium or potassium salt. This may be achieved by adding a suitable hydroxide to the CHD-FA.

The administration would generally be oral. Typically, the CHD-FA, salt or ester thereof would be formulated into a form such as a mouthwash, toothpaste, denture cleanser, impregnated dental floss or dental tape, or endodontic irrigant or the like, with the CHD-FA, salt or ester thereof as the active ingredient, or an adjuvant. Additional ingredients, including flavourants may also be included in the oral formulation.

An *in vitro* study was conducted to evaluate the antimicrobial activity of CDH-FA against a range of oral pathogens associated with caries, periodontal disease, endodontic infection and soft tissue infections. In addition, the toxicological properties of the compound were evaluated.

The following example is for the purpose of illustration only.

### EXAMPLES

### Methods

A panel of oral pathogens, including Gram-positive, Gramnegative and yeasts were grown planktonically and tested with CHD-FA using standardised CLSI methodology. In addition, biofilm populations were grown using a 96-well peg plate method, challenged with CHD-FA, and the sessile MIC's evaluated. Static and cidal activity was assessed. A TR146 oral epithelial cell line was used to assess the toxicity of CHD-FA using both metabolic (XTT) and LDH assays.

### Results

CHD-FA (0.5% active matter) was effective against the entire panel of planktonic Gram positive, Gram negative bacteria and yeasts, which included *Streptococcus mutans, Porphymonas gingivalis* and *Candida albicans,* and it was shown to exhibit cidal activity.

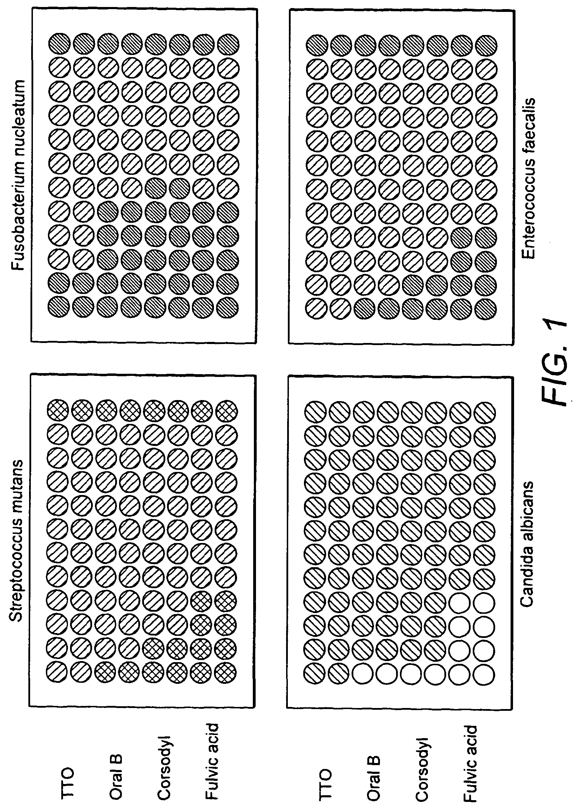

Against biofilms the CHD-FA was less effective, with a range of activity of 2- ≥ 4% active matter. However, CHD-FA was shown to be more effective in killing oral biofilms in comparison to other treatments i.e. Tea Tree Oil and the commercially available products, Oral B and Corsodyl as illustrated in Figure 1.

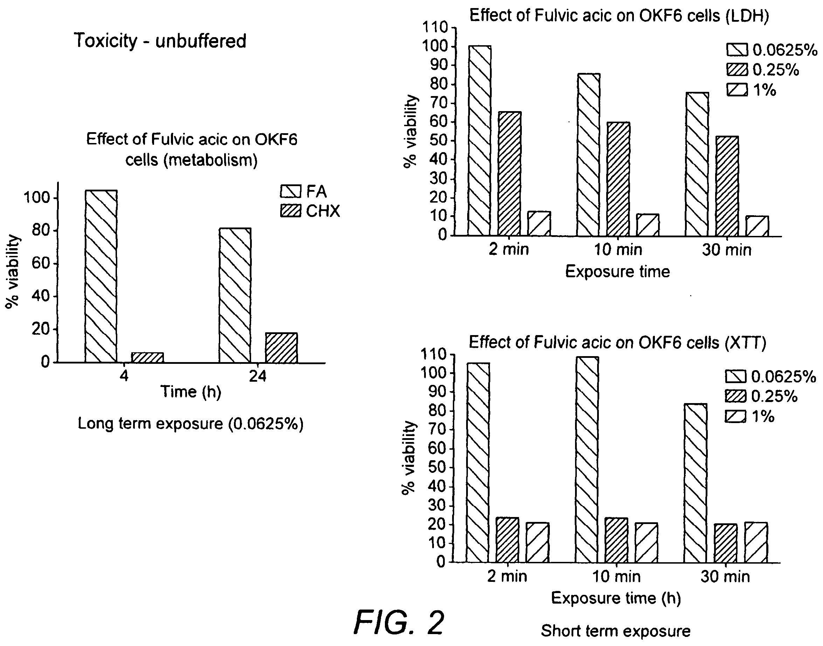

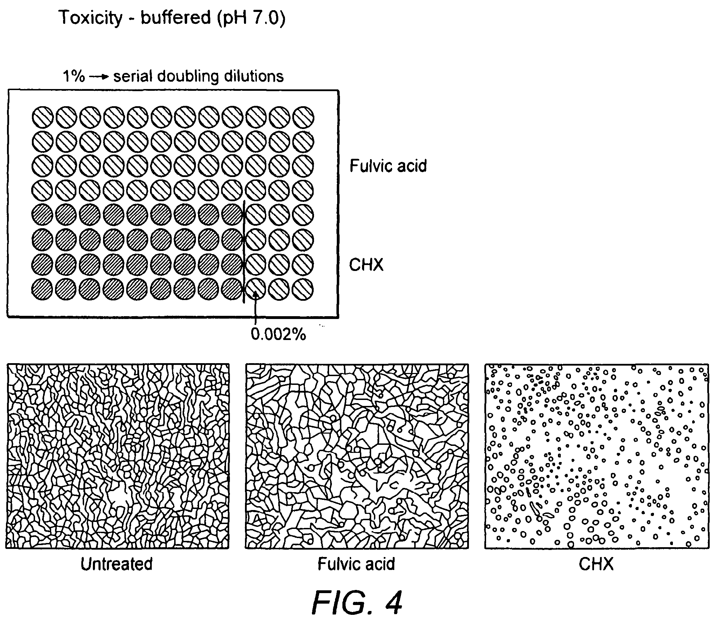

In addition, CHD-FA was shown to be relatively non-toxic when applied to the OKF6 cell line (see Figure 2). When the cell line was exposed a concentration of 0.5% active matter CHD-FA, the planktonic MIC, it did not exhibit any cellular toxicity by either assay. Furthermore, CHD-FA is relatively non-toxic compared to Chlorhexadine, an ingredient commonly used to treat oral pathogens (see Figure 4).

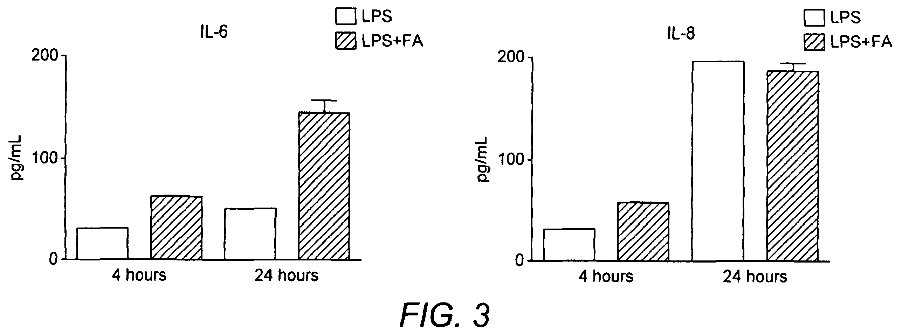

As indicated in Figure 3, CHD-FA was shown to have an immune-modulatory effect on the cytokine IL-6 and the chemokine IL-8 which are often associated with inflammation.

As shown in Table 1, when the efficacy of CHD-FA is compared to the natural product Tea Tree Oil, or existing over-the counter products, Oral B and Corsodyl, we see that CHD-FA is superior to all the other treatments for a range of oral pathogens.

The values in percent in the Figures refers to % active matter.

**Table 1: Efficacy of CHD-FA against a range of oral pathogens**

| | NATURALS | | | | | | OTC's | | | | | |
|---|---|---|---|---|---|---|---|---|---|---|---|---|
| DRUG | Fulvic Acid % | | | Tea Tree Oil % | | | Oral B % | | | Corsodyl® % | | |
| Bacterium | PMIC | PMBC | SMIC | PMIC | PMBC | SMIC | PMIC | PMBC | SMIC | PMIC | PMBC | SMIC |
| S. *sanquinis* | 0.0625 | 0.0625 | 0.25 | >10 | >10 | 10 | 0.3125 | 5 | 10 | 0.3125 | 0.625 | 5 |
| S. *salivarius* | 0.0625 | 0.125 | 0.25 | >10 | >10 | >10 | >10 | >10 | 10 | 0.3125 | 0.3125 | 5 |
| S. *mutans* | 0.125 | 0.25 | 0.25 | >10 | >10 | >10 | 2.5 | >10 | 10 | 0.3125 | 0.625 | 5 |
| E. *faecalis* | 0.125 | 0.25 | 0.25 | >10 | >10 | >10 | 1.25 | 2.5 | 10 | 0.3125 | >10 | 5 |
| *Aggregatibacter actinomycetemcomitans* | 0.0625 | 0.125 | 0.25 | >10 | >10 | >10 | 0.625 | 5 | >10 | 0.625 | 0.625 | 10 |
| F. *nucleatum* | 0.0625 | 0.0625 | 1.25 | >10 | >10 | 5 | 2.5 | 2.5 | 0.625 | 2.5 | 2.5 | 0.3125 |
| *P. gingivalis* | 0.03125 | 0.25 | 0.25 | >10 | >10 | 10 | 10 | >10 | 1.25 | 1.25 | 2.5 | 1.25 |
| C. *albicans* | 0.125 | 0.125 | 0.25 | 0.625 | 2.5 | >10 | 0.156 | 0.156 | 5 | 0.625 | 1.25 | 5 |
| C. *glabrata* | 0.125 | 0.125 | 0.25 | 0.625 | >10 | 0.25 | 0.156 | 0.3125 | 2.5 | 0.625 | 1.25 | 5 |
| C. *tropicalia* | 0.03125 | 0.125 | 0.25 | 0.3125 | >10 | 5 | 0.078 | 0.078 | 2.5 | 0.3125 | 0.3125 | 2.5 |

### Conclusions

This study has shown that the natural antimicrobial CHD-FA is non-toxic and has cidal activity against a range of microbes associated with a variety of oral diseases. Therefore, as a mouthwash, this product has potential as an adjunct to mechanical disruption to minimise the microbial burden in the oral cavity, subject to further studies.

## Claims

1. Carbohydrate-derived fulvic acid (CHD-FA), or a salt or ester thereof as the active ingredient for use in the prevention, treatment or inhibition of infection by an oral pathogen in a mammal, wherein the infection by an oral pathogen is in the form of an oral biofilm.

2. The CHD-FA according to claim 1, wherein the mammal is a human.

3. A composition comprising CHD-FA, or a salt or ester thereof as the active ingredient for use in the prevention, treatment or inhibition of infection by an oral pathogen wherein the infection by an oral pathogen is in the form of an oral biofilm.

4. The composition according to claim 3, in which the CHD-FA, or a salt or ester thereof is present in an amount from about 8% to 60% v/v of the composition.

5. The composition according to claim 4, in which the amount is about 35% v/v of the composition.

6. The composition comprising CHD-FA, or a salt or ester thereof according to claim 3 and a suitable carrier for oral administration.

7. The composition according to claim 6, in the form of a mouthwash.

8. The composition according to claim 6, in the form of a toothpaste.

9. The composition according to claim 6, in the form of impregnated dental floss or dental tape.

10. The composition according to claim 6, in the form of an endodontic irrigant.

11. The composition according to any one of claims 6 to 10, further comprising flavourants.

## Patentansprüche

1. Von einem Kohlenhydrat abgeleitete Fulvinsäure (Carbohydrate-Derived Fulvic Acid, CHD-FA) oder ein Salz oder Ester davon als Wirkstoff zur Verwendung bei der Prävention, Behandlung oder Inhibierung einer Infektion durch ein orales Pathogen bei einem Säugetier, wobei die Infektion durch ein orales Pathogen in Form eines oralen Biofilms vorliegt.

2. CHD-FA nach Anspruch 1, wobei es sich bei dem Säugetier um einen Menschen handelt.

3. Zusammensetzung, umfassend CHD-FA oder ein Salz oder einen Ester davon als Wirkstoff zur Verwendung bei der Prävention, Behandlung oder Inhibierung einer Infektion durch ein orales Pathogen, wobei die Infektion durch ein orales Pathogen in Form eines oralen Biofilms vorliegt.

4. Zusammensetzung nach Anspruch 3, wobei die CHD-FA oder ein Salz oder Ester davon in einer Menge von etwa 8% bis 60% v/v der Zusammensetzung vorliegt.

5. Zusammensetzung nach Anspruch 4, wobei die Menge etwa 35% v/v der Zusammensetzung beträgt.

6. Zusammensetzung, umfassend CHD-FA oder ein Salz oder einen Ester davon nach Anspruch 3 und einen geeigneten Träger für die orale Verabreichung.

7. Zusammensetzung nach Anspruch 6 in Form eines Mundwassers.

8. Zusammensetzung nach Anspruch 6 in Form einer Zahnpasta.

9. Zusammensetzung nach Anspruch 6 in Form von imprägnierter Zahnseide oder imprägniertem Zahnband.

10. Zusammensetzung nach Anspruch 6 in Form einer endodontischen Spülflüssigkeit.

11. Zusammensetzung nach einem der Ansprüche 6 bis 10, die ferner Geschmacksstoffe umfasst.

## Revendications

1. Acide fulvique dérivé d'un hydrate de carbone (CHD-FA), ou un sel ou un ester de celui-ci en tant que principe actif pour son utilisation dans la prévention, le traitement ou l'inhibition d'une infection par un agent pathogène oral chez un mammifère, l'infection par un agent pathogène oral se présentant sous la forme d'un biofilm oral.

2. CHD-FA selon la revendication 1, le mammifère étant un humain.

3. Composition comprenant un CHD-FA ou un sel ou un ester de celui-ci en tant que principe actif pour son utilisation dans la prévention, le traitement ou l'inhibition d'une infection par un agent pathogène oral, l'infection par un agent pathogène oral se présentant sous la forme d'un biofilm oral.

4. Composition selon la revendication 3, dans laquelle le CHD-FA, ou un sel ou un ester de celui-ci est présent en une quantité d'environ 8 % à 60 % volume/volume de la composition.

5. Composition selon la revendication 4, dans laquelle la quantité est d'environ 35 % volume/volume de la composition.

6. Composition comprenant un CHD-FA, ou un sel ou un ester de celui-ci selon la revendication 3 et un véhicule approprié pour une administration orale.

7. Composition selon la revendication 6, sous la forme d'un bain de bouche.

8. Composition selon la revendication 6, sous la forme d'un dentifrice.

9. Composition selon la revendication 6, sous la forme d'un fil dentaire ou d'un ruban dentaire imprégné.

10. Composition selon la revendication 6, sous la forme d'un irrigant endodontique.

11. Composition selon l'une quelconque des revendications 6 à 10, comprenant en outre des aromatisants.
